# EUROPEAN PATENT APPLICATION

(11) **EP 2 607 724 A1**
(43) Date of publication of application: **26.06.2013**
(21) Application number: 12196216.1
(22) Date of filing: 10.12.2012
(51) Int. Cl.: F16C 1/02, D07B 1/06, A61B 17/00

(54) **Wire coil**

(30) Priority: 20.12.2011 JP 2011279058
(71) Applicant: ASAHI INTECC CO., LTD., Nagoya-shi, Aichi 463-0024 (JP)
(72) Inventor: Hashimoto, Takaya, Nagoya-shi, Aichi 463-0024 (JP); Kazahaya , Shingo, Nagoya-shi, Aichi 463-0024 (JP)
(74) Representative: Winter, Brandl, Fürniss, Hübner, Röss, Kaiser, Polte - Partnerschaft

(57) **Abstract**

A wire rope includes a multi-wire coil (10) formed by twisting several metal wires (11) together forming a coil layer of metal wires (11) with gaps (d1, d2)between each adjacent wire (11). In the wire rope, the gaps (d1, d2) are located between the metal wires (11) along an axis of the multi-wire coil (10). Preferably, the gaps (d1, d2) between the metal wires (11) include a first gap (d1) and a second gap (d2) that differs in width.

## Description

### BACKGROUND OF THE INVENTION

### 1. Field of the Invention

The present invention relates to wire ropes.

### 2. Description of the Related Art

Wire ropes, most of which are multi-strand coils formed by twisting multiple wires together, are widely known (see Fig. 7). Japanese Unexamined Patent Application Publication No. 9-49517 discloses a structure of a flexible shaft including a core shaft. In this structure, wires are each helically wound around the core shaft while having minute gaps between turns of the wires. Japanese Unexamined Patent Application Publication No. 2001-280333 discloses a flexible shaft in which at least one of an outer-layer wire and an inner-layer wire is wound while having regular gaps between turns of the wire. Japanese Unexamined Patent Application Publication No. 7-14448 discloses a method of manufacturing a multilayer cable in which multiple core wires serving as an inner layer are twisted together in a direction that is opposite to the direction in which multiple core wires serving as an outer layer are twisted together.

### SUMMARY OF THE INVENTION

However, these widely-known wire ropes, mainly the multi-strand coils, have the following drawbacks. When a force is applied to a wire rope in such a direction as to twist or untwist the wire rope around the axis while the wire rope is bent, the wires are heated by frictional heat due to the wires rubbing against each other or by heat due to plastic deformation. Thus, the wires may become deformed or cut.

The flexible shaft disclosed in Japanese Unexamined Patent Application Publication No. 9-49517 has excellent flexibility since gaps are provided between turns of each wire. However, each of the layers of the flexible shaft is constituted by a single wire. Thus, when the flexible shaft is bent and is twisted around, heat is generated in the shaft and the wires may be cut as a result of the heat generated. Cutting the wires severely degrades the durability of the flexible shaft and the flexible shaft may be broken.

In the flexible shaft disclosed in Japanese Unexamined Patent Application Publication No. 2001-280333, the wires are wound while having gaps between some adjacent turns of the wires in the longitudinal direction. However, the remaining part other than the one wound with the gaps is tightly wound. Since the tightly wound part of the wires of the flexible shaft suffers from heat generated therein, the durability of the whole flexible shaft is not sufficient.

In the cable disclosed in Japanese Unexamined Patent Application Publication No. 7-14448, the wires are each wound with no gaps between turns. Thus, the cable is more likely to suffer from heat and has insufficient durability, as in the case of the flexible shafts described in Japanese Unexamined Patent Application Publication Nos. 9-49517 and 2001-280333.

The present invention is made in view of the above circumstances, and an object of the present invention is to provide a wire rope that has excellent durability and excellent flexibility by preventing wires from rubbing against each other to suppress heat generation.

This object is solved by a wire rope according to claim 1, which includes a multi-strand coil formed by twisting a plurality of metal wires together, wherein the plurality of metal wires forming the first multi-strand coil are twisted so that each two adjacent turns of the metal wires are spaced apart from each other by a gap along an axis of the first multi-strand coil.

By providing gaps between the metal wires in this manner, adjacent turns of metal wires are less likely to interfere with one another and thus the wire rope bends easily. Moreover, if, for example, torque is applied along the axis of the wire rope by bending the wire rope, the metal wires are less likely to come into contact with one another and thus heat generation due to friction can be prevented. Consequently, the wire rope can have a higher durability.

The wire rope according to the present invention has higher durability and excellent flexibility by gaps being provided between turns of each of metal wires to prevent heat generation due to friction between the metal wires when the wires are bent.

The gaps between the metal wires forming the multi-strand coil may be formed over the entire length of the wire rope or may be formed in a, i.e., in at least one, preset section of length of the respective multi-strand coil only. Such preset section of length may form a preset bending point of the wire rope.

### BRIEF DESCRIPTION OF THE DRAWINGS

Fig. 1 schematically illustrates a wire rope according to a first embodiment when viewed in vertical section.
Fig. 2A is a graph showing test results.
Fig. 2B is a schematic diagram of a configuration for the test.
Fig. 3 schematically illustrates a wire rope according to a second embodiment when viewed in vertical section.
Fig. 4 schematically illustrates a wire rope according to a first modification of the second embodiment when viewed in vertical section.
Fig. 5 schematically illustrates a wire rope according to a second modification of the second embodiment when viewed in vertical section.
Fig. 6 schematically illustrates a wire rope according to a third modification of the second embodiment when viewed in vertical section.
Fig. 7 schematically illustrates an existing wire rope when viewed in vertical section.

### DESCRIPTION OF THE PREFERRED EMBODIMENTS

Wire ropes according to embodiments of the present invention will be described referring to the drawings.

### First Embodiment

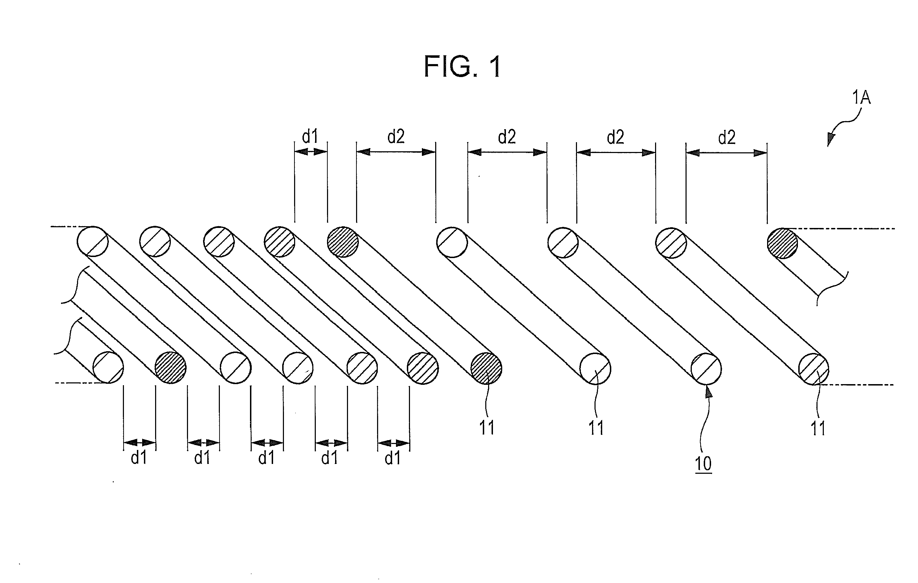

As illustrated in Fig. 1, a wire rope 1A according to a first embodiment of the present invention includes a multi-strand coil 10 formed by twisting multiple metal wires 11 together. The materials of the metal wires 11 are not limited to specific ones, but metals having high hardness, such as stainless steel wires, carbon steel wires, or steel wires for springs, are preferably used.

Gaps are located between the metal wires 11 of the multi-strand coil 10 along the axis of the multi-strand coil 10. To be more specifically, the metal wires 11 forming the multi-strand coil 10 are all twisted so that each two adjacent turns of the metal wires 11 are spaced apart from each other by a gap along a longitudinal axis of the multi-strand coil 10 or wire rope 1A. Furthermore, in this embodiment the twisted metal wires 11 of the multi-strand coil 10 are all arranged in a cylindrical layer to form a tubular multi-strand coil body. Accordingly, the metal wires 11 are all arranged without touching each other.

Here, the gaps between the metal wires 11 include gaps d1 and d2 that differ in width. Specifically, the gaps between the metal wires 11 include narrow gaps d1 and wide gaps d2, which are wider than the narrow gaps d1.

As described above, locating gaps between the metal wires 11 improves the flexibility of the wire rope 1A. This is because, when the wire rope 1A is bent, the metal wires 11 easily approach one another without interfering with one another at the inner side of the bent portion. If, for example, torque is applied along the axis of the wire rope 1A by bending the wire rope 1A, the metal wires 11 are less likely to come into contact with one another. This prevents heat generation due to friction between the metal wires 11 and prevents the metal wires 11 from being cut or suffering from other defects. Thus, the durability of the wire rope 1A improves.

The gaps between the metal wires 11 include the narrow gaps d1 and the wide gaps d2 that differ in width. Thus, if the wire rope 1A is bent into various shapes, the metal wires 11 can be flexibly deformed in accordance with these shapes. Thus, the wire rope 1A has a favorable flexibility and heat generation due to friction can be reliably prevented.

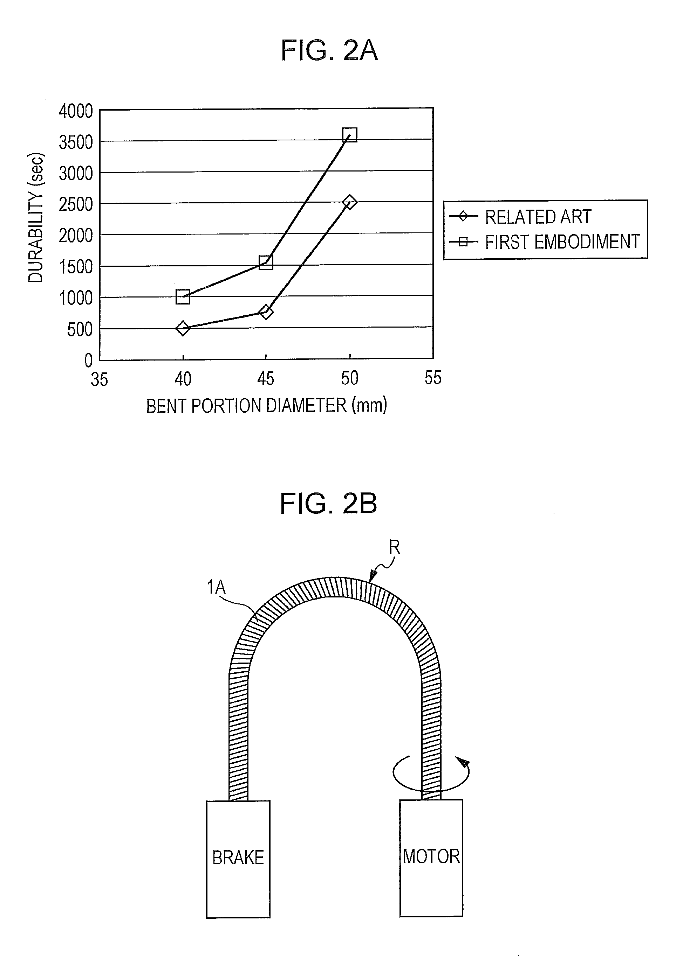

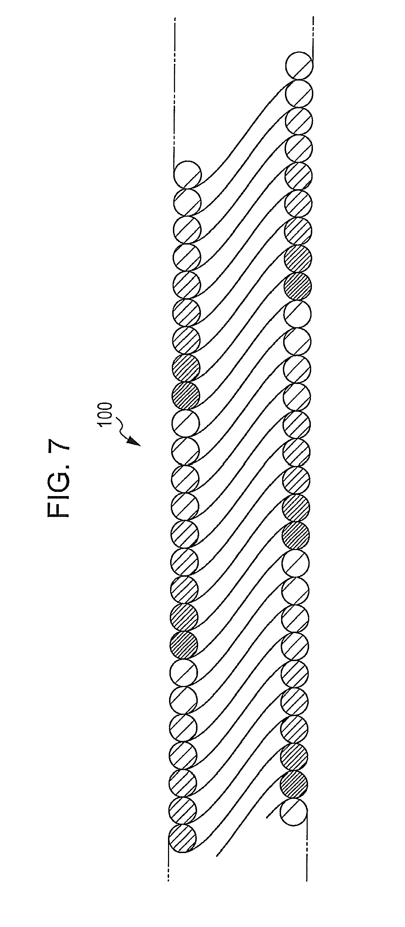

Referring now to Figs. 2A and 2B, a durability test for wire ropes and the results of the test will be described. Wire ropes 1A according to the first embodiment and wire ropes 100 according to a related art (see Fig. 7) are used for the test. In the test, the durability of the wire ropes was measured under the following conditions. As illustrated in Fig. 2B, the wire ropes were bent, one end of each wire rope was connected to a motor, and the other end of the wire rope was connected to a brake. The motor was driven in order to consecutively apply torque along the axis of the wire rope. Tests were conducted under the following conditions: the diameters of bent portions R were set at 40 mm, 45 mm, and 50 mm; the rotational speed of the motor was 1800 (rpm); and the braking force was 20 (N·mm). The duration until an unusual sound came from the tested wire rope was determined as the durability (sec).

Fig. 2A shows the test results. The durability of each wire rope 1A according to the first embodiment and the durability of each wire rope 100 according to the related art were both increased as the diameter of the bent portion R of the tested wire rope became larger. The durability of the wire ropes 1A according to the first embodiment was approximately 200%, at maximum, of the durability of the corresponding wire ropes 100 according to the related art. The durability of the wire ropes 1A is probably improved by the following reasons. The metal wires 11 of the wire ropes 1A are less likely to rub against each other, heat generation due to friction between the metal wires 11 can be prevented, and thus the metal wires 11 are prevented from being cut.

The wire rope 1A may include gaps of the uniform width between the metal wires 11. However, in order for the metal wires 11 to be flexibly deformable in accordance with various shapes into which the wire rope 1A is bent, it is preferable that the gaps between the metal wires 11 include the narrow gaps d1 and the wide gaps d2 that differ in width.

The wire rope 1A illustrated in Fig. 1 has a portion in which multiple narrow gaps d1 are successively formed and a portion in which multiple wide gaps d2 are successively formed. However, this is not the only limitation. The narrow gaps d1 and the wide gaps d2 may be alternately formed or may be formed at random.

From the view point of flexibility of the metal wires 11 with which the metal wires 11 are deformable in accordance with various shapes into which the wire rope 1A is bent, it is preferable that the narrow gap d1 and the wide gap d2 are formed alternately or at random. In the first embodiment, other gaps whose width differs from those of the narrow gaps d1 and the wide gaps d2 may be further provided between the metal wires 11.

### (Second Embodiment)

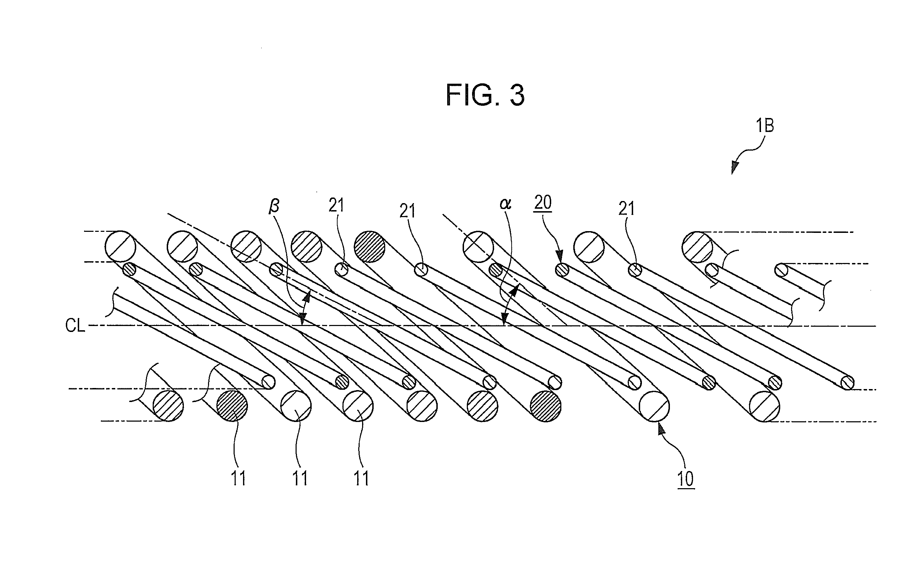

Referring to Fig. 3, a second embodiment will be described now. Portions that are the same as those in the first embodiment will not be described and the same portions are denoted by the same reference symbols.

As illustrated in Fig. 3, a wire rope 1B according to a second embodiment of the present invention includes a multi-strand coil 10 and a multi-strand coil 20 that is disposed inside the multi-strand coil 10. Hereinbelow, the multi-strand coil 10 disposed on the outer side is referred to as an outer multi-strand coil 10, and the multi-strand coil 20 disposed on the inner side is referred to as an inner multi-strand coil 20.

The outer multi-strand coil 10 corresponds, as regards material, structure and arrangement, to the multi-strand coil 10 of the first embodiment. Like the outer multi-strand coil 10, the inner multi-strand coil 20 may be formed by twisting a plurality of metal wires 21 together with gaps formed between each other along an axis of the inner multi-strand coil 20. In other words, the metal wires 21 of the inner multi-strand coil 20 may all be twisted so that each two adjacent turns of the metal wires 21 are spaced apart from each other by a gap along a longitudinal axis of the inner multi-strand coil 20 or wire rope 1B. The materials of the metal wires 21 are not limited to specific ones, but metals having high hardness, such as stainless steel wires, carbon steel wires, or steel wires for springs, are preferably used.

The outer multi-strand coil 10 is wound around the outer circumference of the inner multi-strand coil 20 without a space being provided between the inner circumference of the outer multi-strand coil 10 and the outer circumference of the inner multi-strand coil 20. In this embodiment, the twisted metal wires 21 of the inner multi-strand coil 20 are all arranged in a cylindrical layer of a smaller diameter than the metal wires 11 of the outer multi-strand coil 10. Further, the multi-strand coils 10 and 20 are arranged such that the outer multi-strand coil 10 is wound in the same direction as the inner multi-strand coil 20. A winding angle α, at which the outer multi-strand coil 10 is wound when viewed in vertical section, is made different from a winding angle β, at which the inner multi-strand coil 20 is wound when viewed in vertical section. Specifically, the winding angle α is formed between the center line CL of the wire rope 1B when viewed in vertical section and the center line of each metal wire 11 of the outer multi-strand coil 10, and the winding angle β is formed between the center line CL of the wire rope 1B when viewed in vertical section and the center line of each metal wire 21 of the inner multi-strand coil 20.

As described above, winding the outer multi-strand coil 10 and the inner multi-strand coil 20 in the same direction negligibly distorts the multi-strand coils 10 and 20 when torque is applied to the wire rope 1B. Thus, the entirety of the wire rope 1B has a higher torque resistance. In addition, the multi-strand coils 10 and 20 are in point contact with one another since the winding angle α of the outer multi-strand coil 10 and the winding angle β of the inner multi-strand coil 20 are different from each other. Consequently, the metal wires 21 of the inner multi-strand coil 20 are prevented from entering the gaps between the metal wires 11 of the outer multi-strand coil 10. In this embodiment, the winding angle α of the outer multi-strand coil 10 is larger than the winding angle β of the inner multi-strand coil 20. Thus, tension in the longitudinal direction of the metal wires 11 of the outer multi-strand coil 10 can be reduced. This can reduce fatigue of the metal wires 11 of the outer multi-strand coil 10 and the wire rope 1B can have a higher durability.

A first modification of the second embodiment will be described now.

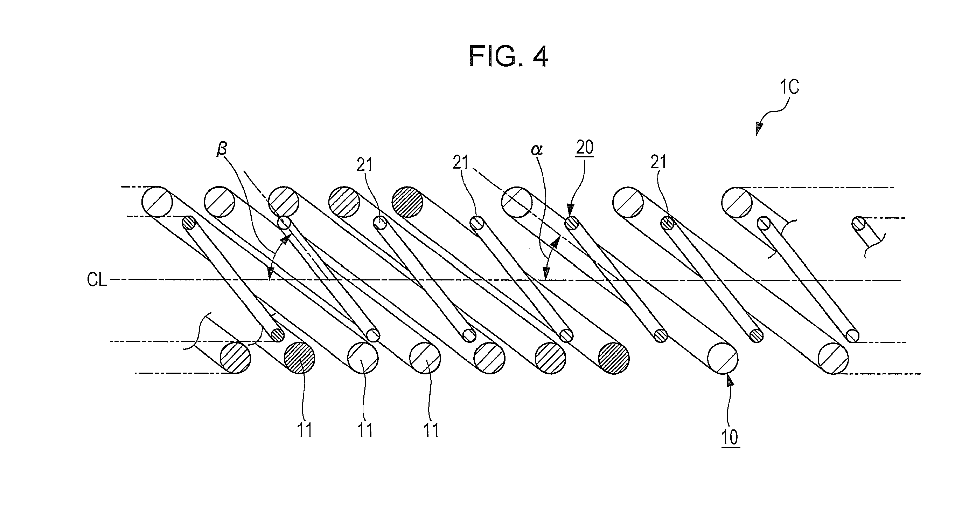

As illustrated in Fig. 4, a wire rope 1C according to the first modification includes an outer multi-strand coil 10 and an inner multi-strand coil 20, which are wound in the same direction. The winding angle α of the outer multi-strand coil 10 when viewed in vertical section is smaller than the winding angle β of the inner multi-strand coil 20 when viewed in vertical section.

If torque is applied to the wire rope 1C having the above-described configuration, the multi-strand coils 10 and 20 are less likely to be distorted and the entirety of the wire rope 1C has a higher torque resistance. In addition, since the multi-strand coils 10 and 20 are in point contact with one another, the metal wires 21 of the inner multi-strand coil 20 are prevented from entering the gaps between the metal wires 11 of the outer multi-strand coil 10.

A second modification of the second embodiment will be described now.

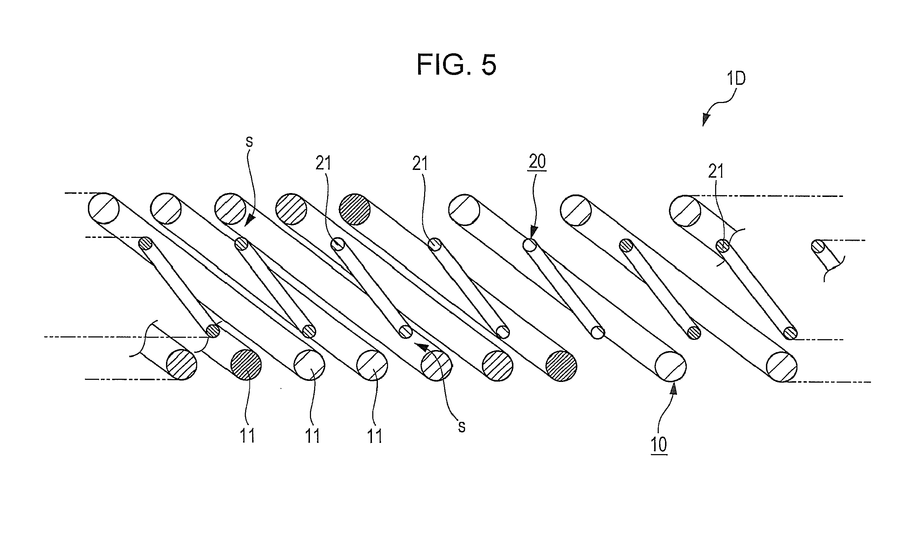

As illustrated in Fig. 5, in a wire rope 1D according to the second modification, a space s is provided between the inner circumference of an outer multi-strand coil 10 and the outer circumference of an inner multi-strand coil 20.

The wire rope 1D having this configuration can be easily bent because metal wires 11 of the outer multi-strand coil 10 and metal wires 21 of the inner multi-strand coil 20 are less likely to interfere with one another. If torque is applied along the axis of the wire rope 1D by bending the wire rope 1D, heat generation due to friction is more reliably prevented. Thus, the wire rope 1E can have a higher durability.

A third modification of the second embodiment will be described now.

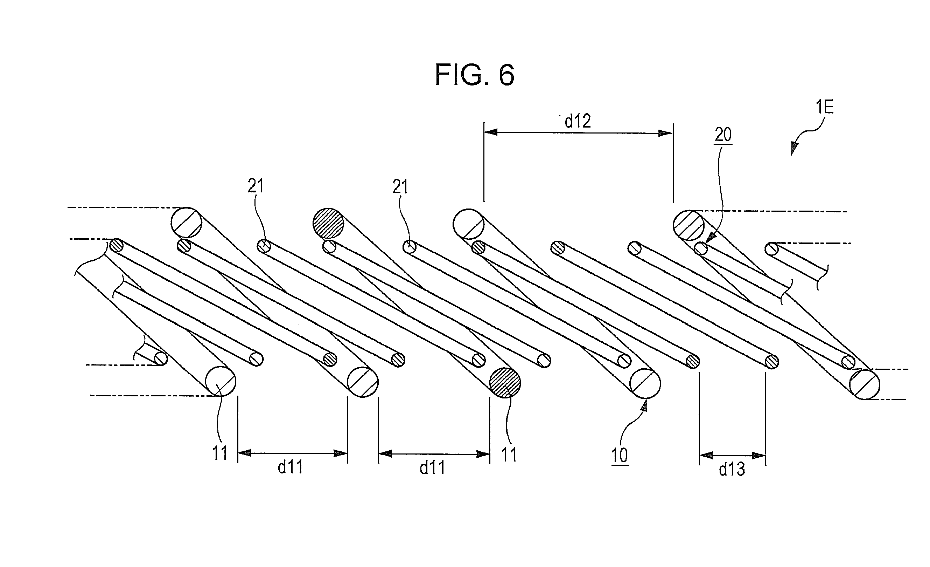

As illustrated in Fig. 6, in a wire rope 1E according to the third modification, gaps d11 and d12 between metal wires 11 of an outer multi-strand coil 10 are wider than gaps d13 between metal wires 21 of an inner multi-strand coil 20.

The wire rope 1E having this configuration is more easily bent. If torque is applied along the axis of the wire rope 1E by bending the wire rope 1E, heat generation due to friction can be more reliably prevented. Thus, the wire rope 1E can have a higher durability.

The present invention is not limited to the above-described embodiments and the embodiments may be modified or combined as appropriate within a scope not departing from the gist of the invention.

In particular, the gaps d1, d2, d11, and d12 between the metal wires 11 forming the multi-strand coil 10 and/or the gaps d13 between the metal wires 21 forming the multi-strand coil 20 may each be formed over the entire length of the wire rope as in the above described embodiments, or may each be formed in at least one preset section of length of the respective multi-strand coil only. Such preset section of length may form a preset bending point of the wire rope. For example, the gaps d1, d2, d11, and d12 between the metal wires 11 and/or the gaps d13 between the metal wires 21 may be provided at least at main portions of the wire ropes 1A to 1E, the main portions being deformable into curves in the longitudinal direction.

The wire ropes 1A to 1E are each preferably usable as a component of tools, such as an endoscopic surgical tool in which a driving core shaft is inserted into the outer multi-strand coil 10 and the inner multi-strand coil 20, or as a component of an industrial component.

## Claims

1. A wire rope (1A, 1B, 1C, 1D, 1E) comprising:
a first multi-strand coil (10) formed by twisting a plurality of metal wires (11) together, **characterized in that**
the plurality of metal wires (11) forming the first multi-strand coil (10) are twisted so that each two adjacent turns of the metal wires (11) are spaced apart from each other by a gap along an axis of the first multi-strand coil (10).

2. The wire rope (1A, 1B, 1C, 1D, 1E) according to Claim 1, wherein the gaps (d1, d2; D11, D12) between the plurality of metal wires (11) include a first gap (d1; D11) and a second gap (d2; D12) that differ in width.

3. The wire rope (1B, 1C, 1D, 1E) according to Claim 1 or 2, further comprising:
a second multi-strand coil (20),
wherein the second multi-strand coil (20) is disposed inside the first multi-strand coil (10) such that the first multi-strand coil (10) on an outer side is wound around an outer circumference of the second multi-strand coil (20) on an inner side, and
wherein the first multi-strand coil (10) on the outer side is wound in the same direction as the second multi-strand coil (20) on the inner side, and an angle (α) at which the first multi-strand coil (10) on the outer side is wound when viewed in vertical section differs from an angle (β) at which the second multi-strand coil (20) on the inner side is wound when viewed in vertical section.

4. The wire rope (1B, 1C, 1D, 1E) according to Claim 3, wherein the angle at which the first multi-strand coil (10) on the outer side is wound when viewed in vertical section is larger than the angle at which the second multi-strand coil (20) on the inner side is wound when viewed in vertical section.

5. The wire rope (1B, 1C, 1D, 1E) according to Claim 3 or 4, wherein in at least the preset section of length of the wire rope (1B, 1C, 1D, 1E) the plurality of metal wires (21) of the second multi-strand coil (20) are twisted so that each two adjacent turns of the metal wires (11) are spaced apart from each other by a gap along an axis of the second multi-strand coil (10).
